# FASCICULE DE BREVET EUROPEEN

(11) **EP 0 745 377 B1**
(45) Date de publication et mention de la délivrance du brevet: **11.06.1997**
(21) Numéro de dépôt: 96401076.3
(22) Date de dépôt: 15.05.1996
(51) Int. Cl.: A61K 7/48, A61K 7/06, A61K 7/08, A61K 7/02

(54) **Composition solide expansée formé à partir d'un produit naturel par utilisation en cosmétique**
Expandiertes festes Produkt auf Basis von natürlichen Stoffe zur Verwendung in Kosmetik
Natural product-based expanded solid composition for use in cosmetics

(30) Priorité: 29.05.1995 FR 9506320; 16.06.1995 FR 9507247
(43) Date de publication de la demande: 04.12.1996
(73) Titulaire: L'OREAL, 75008 Paris (FR)
(72) Inventeur: Roulier, Véronique, 75002 Paris (FR); Mellul, Myriam, 94240 L'Hay-Les-Roses (FR); Gabin, Gérard, 75008 Paris (FR); Holz, Katrin, 1012 Lausanne (CH)
(74) Mandataire: Miszputen, Laurent

(56) Documents cités:
- EP-A- 0 515 246
- EP-A- 0 605 284
- FR-A- 1 594 256

## Description

La présente invention concerne l'utilisation comme produits cosmétiques de compositions solides expansées dont la matrice est constituée d'un réseau alvéolaire formé à partir d'un produit riche en amidon ou d'un dérivé de produit riche en amidon apte à s'expanser ainsi que les compositions cosmétiques et dermatologiques mises en oeuvre.

On connaît dans l'industrie alimentaire des produits expansés à base de substances naturelles ou de dérivés desdites substances aptes à s'expanser, notamment l'amidon, et d'ingrédients comestibles, obtenus par extrusion dans un ou plusieurs extrudeurs mono- ou bi-vis notamment les snacks apéritifs, les chips, les cornflakes, les produits pour petit-déjeuner à base de céréales, les biscuits.

Le document EP-A-0 515 246 décrit des microparticules coagulées de protéines globulaires obtenues par extrusion dans un extruder bi-vis et leur utilisation dans des formulations de texture onctueuse, alimentaires ou cosmétiques, à base de caséinates.

La demanderesse a découvert de manière surprenante de nouvelles compositions à usage cosmétique ou dermatologique sous la forme d'une composition solide expansée dont la matrice est constituée d'un réseau alvéolaire formé à partir d'un produit riche en amidon ou d'un dérivé de produit riche en amidon apte à s'expanser.

Les compositions selon l'invention peuvent contenir des charges minérales et/ou des charges organiques. Elles peuvent constituer en elles-mêmes de nouvelles formes de produits de maquillage tels que des poudres visages, des fards à paupières ou des fards à joues, de nouvelles formes de produit pour l'hygiène tels que des shampooings secs ou pour le soin tels que des produits de démaquillage. Elles présentent l'aspect de boudins, de pellets, de feuilles ou de flocons expansés, pouvant contenir suffisamment de charges pour obtenir un bon délitage et des qualités de douceur satisfaisantes.

Elles peuvent en outre contenir des quantités importantes de phase grasse permettant d'améliorer le confort et de faciliter leur application sur la peau, par exemple, en pouvant les appliquer directement sans l'intermédiaire d'un outil de maquillage (pinceau, éponge, houppette).

La demanderesse a découvert de façon inattendue que l'introduction de fortes quantités de charges dans une matrice expansée formée à partir d'un produit riche en amidon ou d'un dérivé de produit riche en amidon apte à s'expanser, ne diminuait pas de manière significative le taux d'expansion et permettait l'introduction de fortes quantités de corps gras tels que des huiles et/ou des cires.

Les compositions selon l'invention peuvent notamment contenir, dans des quantités importantes, des cires conférant des propriétés de tenue de film, de glissant et de mat. Les poudres compactées habituellement utilisées pour le maquillage ne peuvent pas contenir des quantités importantes de corps gras tels que des cires (plus de 10% en poids). Leur incorporation dans des poudres conduit à des produits qui cirent et qui ne peuvent être délités.

Les compositions selon l'invention peuvent de plus contenir des quantités importantes de charges difficilement compactables conférant un toucher très doux et non-gras. Par charge difficilement compactable, on entend une matière première qui, à partir d'un certain pourcentage qui dépendra de la matière en question, ne peut être compactée au moyen d'une presse mécanique. Ces types de charge ne peuvent pas être utilisées à des concentrations importantes dans les produits de maquillage sous forme de poudre compactée. De plus, les produits de maquillage les contenant ne présentent pas une bonne intégrité au stockage, une bonne solidité aux chocs et/ou une surface plane convenable.

Les compositions de l'invention par leur nouvelle structure alvéolaire peuvent constituer de nouvelles formes galéniques solides pour le maquillage sous forme de boudins, pellets, feuilles ou flocons expansés appliqués directement sur la peau ou le visage ou être réduites en poudre et utilisées de façon classique comme poudre de maquillage sans présenter les inconvénients évoqués ci-dessus.

Elles peuvent se présenter sous forme de poudre de soin et/ou d'hygiène, appliquée directement sur la peau, le cuir chevelu ou les cheveux par exemple des shampooings à sec ou des poudres libres pour le soin du corps.

Elles peuvent également se présenter sous forme de boudins, de pellets, de feuilles ou de flocons expansés ou bien de poudre, stockés à l'état sec, et très facilement réhydratables après immersion dans un milieu aqueux pour reconstituer des formulations pour le maquillage telles que des fonds de teint ou des formulations pour le soin ou l'hygiène telles que des crèmes, des laits, des bains moussants, des gels, des shampooings. Il est ainsi possible d'incorporer dans ces formes galéniques, stables au stockage à des températures inférieures à 45°C, des actifs cosmétiques hydrosensibles.

Les compositions selon l'invention, stockées à sec et destinées à être réhydratées au moment de l'emploi pour reconstituer les formulations cosmétiques telles que citées ci-dessus, présentent l'avantage par rapport aux formes galléniques réhydratables classiques, d'être très facilement réhydratable et dans le cadre des compositions de nettoyage, notamment les shampooings, d'être moins agressive du fait que les tensio-actifs sont intégrés dans une matrice expansée.

Réduites à l'état de poudre, elles peuvent également être utilisées comme phase pulvérulente dans la fabrication de produits de maquillage et en particulier des poudres compactées et conduire à des poudres plus légères, plus homogènes, de toucher très doux et non-gras, sans les inconvénients évoqués ci-dessus.

Les compositions selon l'invention sont des compositions solides expansées dont la matrice est constituée d'un réseau alvéolaire formé à partir d'un produit naturel ou d'un dérivé de produit naturel apte à s'expanser et contient au moins une substance cosmétique ou dermatologique.

Les produits riches en amidon ou leurs dérivés formant la matrice expansée des compositions selon l'invention sont choisis de préférence parmi ceux aptes à s'expanser par un procédé d'extrusion.

Parmi les produits naturels et leurs dérivés aptes à s'expanser utilisés selon l'invention, on peut citer l'amylose et/ou l'amylopectine et les produits riches en amidon contenant ces deux constituants ; ainsi que leurs dérivés.

Les produits riches en amidon sont préférentiels. Ils sont choisis en particulier parmi les farines de céréale telles que les farines de blé, de maïs, de riz, d'avoine, de germes de blé ou la farine de pomme de terre ; les amidons purs couramment utilisés en alimentaire tels que les amidons de maïs, de pomme de terre, de tapioca, d'avoine ; les amidons modifiés au niveau du rapport amylose/amylopectine tels que le produit HYLON VII vendu par la société AMYLUM ; les amidons modifiés par réticulation ou par un groupe fonctionnel tels que l'amidon de maïs réticulé vendu sous le nom RESISTAMYL E2 par la société AMYLUM, l'amidon de maïs faiblement quaternisé vendu sous le nom MYPLUS W7 par la société AMYLUM, l'amidon de pomme de terre vendu sous le nom SUPRAMYL P 60 par AMYLUM ou l'amidon de maïs hydroxypropylé vendu sous le nom MERIGEL EF6 par AMYLUM.

Les compositions selon l'invention contiennent de préférence au moins une charge minérale et/ou une charge organique.

Les charges utilisées dans les compositions de l'invention sont des particules cosmétiques ou dermatologiques insolubles dans le milieu formé par la matrice amidonnée.

Elles sont présentes dans les compositions de l'invention, à des concentrations allant de 2 à 75% en poids par rapport au poids total de la composition.

Lorsque les charges ont une densité très faible, notamment inférieure à 0,1 g.cm⁻³, elles sont de préférence présentes à raison de 2-20% en poids par rapport à la composition finale.
Lorsque les charges ont une densité plus élevée, notamment supérieure à 0,5 g.cm⁻³, elles sont de préférence présentes à raison de 10-75% en poids par rapport à la composition finale.

Les charges utilisées selon l'invention sont choisies de préférence parmi les charges minérales ou organiques, de structure lamellaire ou sphérique ou leurs mélanges. Elles peuvent être compactables ou difficilement compactables.

Chaque type de charges permet d'apporter des qualités particulières et différentes à la composition selon l'invention. Ainsi, par exemple, les charges de type lamellaires minérales apportent généralement de la douceur, les charges de type sphériques minérales apportent généralement un bon délitage et les charges sphériques organiques ont généralement un rôle structurant et apportent de la douceur.

Parmi les charges de type minérales lamellaires, on peut citer :
- les talcs ou silicates de magnésium hydratés, sous forme de particules de dimensions généralement inférieures à 40 µm ;
- les micas ou aluminosilicates de compositions variées et qui se présentent sous forme d'écailles ayant des dimensions de 2 à 200 µm, de préférence 5-70 µm et une épaisseur de 0,1 à 5 µm, de préférence de 0,2-3 µm, ces micas pouvant être d'origine naturelle (par exemple muscovite, margarite, roscoelite, lipidolite, biotite) ou d'origine synthétique. Ils sont généralement transparents et permettent de conférer à la peau un aspect satiné ;
- les argiles telles que les séricites, qui appartiennent à la même classe chimique et cristalline que la muscovite mais dont les propriétés organoleptiques sont proches du talc ;
- le kaolin ou silicate d'aluminium hydraté, qui se présente sous la forme de particules de formes isotropes ayant des dimensions généralement inférieures à 30µm et qui possèdent de bonnes propriétés d'absorption des corps gras ;
- les nitrures de bore.

Ces charges sont généralement compactables.

Toutefois, parmi ces charges de type lamellaires minérales, certaines sont difficilement compactables. On peut ainsi citer :
- certains talcs, tels que le 'Talc K1' de la société NIPPON ou le 'Talc Extra Steamic OOS' de la société LUZENAC ;
- certaines séricites, telles que la 'Séricite BC282' de la société WHITTAKER ;
- la plupart des micatitanes lorsqu'on les utilise à un fort pourcentage, parmi lesquels on peut citer le mica-nanotitane 'Coverleaf PC 2055M' de la société IKEDA.

Parmi les charges de type lamellaires organiques compactables, on peut citer les poudres de polymères de tétrafluoroéthylène, telles que le 'Fluon' de la société MONTEFLUOS, ou le 'Hostaflon' de la société HOECHST.

Parmi les charges de type lamellaires organiques difficilement compactables, on peut citer la lauroyl-lysine 'Aminhope LL-11' de la société AJINOMOTO.

Parmi les charges de type sphériques minérales compactables, on peut citer :
- les oxydes de zinc et de titane, généralement utilisés sous la forme de particules ayant des dimensions ne dépassant pas quelques micromètres (ou même inférieures à 1 µm dans le cas de l'oxyde de titane), en particulier des dioxydes de titane sphériques comme le 'SPHERITITAN' de la société IKEDA; ces oxydes ont un toucher onctueux, un bon pouvoir couvrant et une opacité importante ;
- le carbonate de calcium précipité qui, sous forme de particules de dimensions supérieures à 10 µm, a un toucher onctueux et permet d'obtenir un aspect mat ;
- le carbonate et l'hydrocarbonate de magnésium, qui possèdent, notamment, des propriétés de fixation des parfums ;
- la silice sphérique non poreuse et
- l'hydroxyapatite.

Parmi les charges de type minérales sphériques difficilement compactables, on peut citer :
- les microsphères de silice à porosité ouverte ou, de préférence, les microsphères de silice creuses, telles que les 'SILICA BEADS' de la société MAPRECOS, ces microsphères étant avantageusement imprégnées d'un actif cosmétique, et
- les microcapsules de verre ou de céramique 'MACROLITE' de la société 3M.

Parmi les charges de type organiques sphériques compactables, on peut citer :
- les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; ces savons, présents généralement sous la forme de particules ayant des dimensions inférieures à 10 µm, ont un toucher onctueux et facilitent l'adhérence de la poudre à la peau ;
- les poudres-de polymères synthétiques non expansés, tels que le polyéthylène, les polyesters (par exemple isophtalate ou téréphtalate de polyéthylène) et les polyamides (par exemple le Nylon), sous la forme de particules ayant des dimensions inférieures à 50 µm, qui possèdent des propriétés absorbantes et permettent de conférer à la peau un aspect velouté ;
- les poudres de polymères synthétiques, réticulés ou non, sphéronisées comme les poudres d'acide polyacrylique ou polyméthacrylique, les poudres de polystyrène réticulé par le divinylbenzène et les poudres de résine de silicone, et
- des poudres de matériaux organiques d'origine naturelle comme l'octenylsuccinate d'amidon vendu sous le nom DRY FLOW PLUS par la société AMYLUM.

Parmi les charges de type organiques sphériques difficilement compactables, on peut citer:
- les microsphères microporeuses de polymères, qui ont une structure analogue à celle d'une éponge ; elles ont, en général, une surface spécifique d'au moins 0,5 m²/g et, en particulier, d'au moins 1 m²/g, ladite surface spécifique n'ayant pas de limite supérieure autre que celle résultant de la possibilité pratique de réaliser des microsphères de porosité très élevée : la surface spécifique peut, par exemple, atteindre 1 000 m²/g ou même davantage. On peut citer les microsphères de polymères acryliques, telles que celles en copolymère d'acrylate réticulé 'Polytrap' de la société DOW CORNING, et celles de polyméthacrylate de méthyle 'MICROPEARL M' ou 'MICROPEARL M 100' de la société SEPPIC ; ces microsphères microporeuses peuvent avantageusement être imprégnées, notamment par des actifs cosmétiques : on peut citer, à cet égard, les microsphères de copolymères de styrène-divinylbenzène vendues sous la dénomination commerciale 'PLASTIC POWDER FPSQ' par la société TOSHIKI, qui sont imprégnées de squalane qui est un actif cosmétique émollient ;
- les microcapsules de polymères ; qui comportent une seule cavité fermée et forment un réservoir, qui peut contenir un liquide, notamment un actif cosmétique ; elles sont préparées par des procédés connus tels que ceux décrits dans le brevet US-A 3 615 972 et EP-A 0 56219. Elles peuvent être réalisées, par exemple, en polymères ou copolymères d'acides, d'amines ou d'esters monomères à insaturation éthylénique, en polymères urée-formaldéhyde, en polymères ou copolymères de chlorure de vinylidène ; à titre d'exemple, on peut citer les microcapsules faites de polymères ou copolymères d'acrylate ou de méthacrylate de méthyle, ou encore de copolymères de chlorure de vinylidène et d'acrylonitrile ; parmi ces derniers, on indiquera, notamment, ceux qui contiennent, en poids 20-60% de motifs dérivés de chlorure de vinylidène, 20-60% en poids de motifs dérivés d'acrylonitrile et 0-40% en poids d'autres motifs tels que des motifs dérivés d'un monomère acrylique et/ou styrénique ; on peut également utiliser des polymères ou copolymères acryliques réticulés, par exemple dans le cas de polymères comportant un groupement carboxylique, par des diols servant d'agents réticulants; à titre d'exemple, on peut citer les microcapsules en copolymère de chlorure de vinylidène-acrylonitrile 'EXPANCEL' de la société Casco Nobel, les microcapsules 'Q-MAX' de la société Q-MAX et les microcapsules '3 M' de la société 3M.

La matrice constituée du réseau alvéolaire formé à partir d'un produit riche en amidon ou d'un dérivé de produit riche en amidon apte à s'expanser est présente dans les compositions selon l'invention, de préférence, dans une proportion allant de 25 à 98% en poids par rapport au poids de la composition.

Lorsque les charges ont une densité très faible, notamment inférieure à 0,1 g.cm⁻³, la matrice expansée amidonnée représente, de préférence, 80 à 98% du poids total de la composition finale.

Lorsque les charges ont une densité plus élevée, notamment supérieure à 0,5 g.cm⁻³, la matrice expansée amidonnée représente, de préférence, 25 à 90% du poids total de la composition finale.

Les compositions selon l'invention présente une teneur en eau de préférence inférieure ou égale à 5% en poids et plus particulièrement allant de 1 à 2% en poids par rapport au poids de la composition.

Les compositions selon l'invention peuvent contenir en plus une phase grasse. Cette phase grasse peut comprendre des huiles et/ou des cires d'origine animale, végétale, minérale ou synthétique, seules ou en mélanges.

Parmi les huiles utilisables, on peut citer l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; les huiles d'hydrocarbures, telles que les huiles de paraffine, le squalane, la vaseline ; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyl-décyle, le palmitate de 2-octyl-décyle, le myristate ou le lactate de 2-octyldodécyle, le succinate de 2-éthyl-hexyle, le malate de diisostéaryle, le triisostéarate de glycérine ou de diglycérine ; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique.

Parmi les cires utilisables, on peut citer les cires d'abeille, les cires de lanoline et les cires d'insectes de Chine ; les cires de Carnauba, de Candelila, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée ; les paraffines, les cires microcristallines, les cires de Montan et les ozokérites ; les cires de polyéthylène, les cires obtenues par la synthèse de Fisher-Tropsch, les copolymères cireux ainsi que leurs esters, et les cires de silicone telles que les polyalcoxy et polyalkylsiloxanes.

La phase grasse est présente dans des proportions allant, préférentiellement, de 2 à 30% en poids et plus particulièrement de 5 à 15% en poids par rapport au poids total de la composition.

La phase grasse peut, en outre, comprendre des additifs tels que des actifs cosmétiques lipophiles et/ou des ingrédients liposolubles généralement utilisés en cosmétique comme les parfums. De préférence, ces additifs peuvent être présents en une quantité de 0-20% par rapport au poids total de la phase grasse.

Les compositions selon l'invention peuvent contenir, outre les charges, des pigments, de préférence en une quantité de 0-50% par rapport au poids total de la composition finale. Ces pigments peuvent être choisis parmi les pigments minéraux, les pigments organiques et les pigments nacrés.

Parmi les pigments minéraux, on peut citer, par exemple le dioxyde de titane (rutile ou anatase), éventuellement traité en surface; les oxydes de fer noir, jaune, rouge et brun ; le violet de manganèse; le bleu outremer ; l'oxyde de chrome éventuellement hydraté ; le bleu ferrique.

Parmi les pigments organiques, on peut citer, par exemple, les pigments D & C red, D & C orange, D & C yellow, le noir de carbone, et les laques à base de carmin de cochenille.

Les pigments nacrés peuvent être choisis, notamment, parmi les pigments nacrés blancs tels que le mica recouvert d'oxyde de titane ou l'oxychlorure de bismuth; les pigments nacrés colorés tels que le micatitane avec des oxydes de fer, le micatitane avec du bleu ferrique ou de l'oxyde de chrome, le micatitane avec un pigment organique du type précité, ainsi que les pigments à base d'oxychlorure de bismuth.

Les compositions selon l'invention peuvent également contenir un ou plusieurs tensio-actifs non ioniques, anioniques, cationiques ou amphotères, habituellement utilisés en cosmétique. La quantité d'agent tensioactif utilisée est de préférence de 2 à 30% par rapport au poids total de la composition.

Les compositions selon l'invention peuvent également contenir en plus des actifs cosmétiques hydrosolubles.

Parmi les actifs cosmétiques, on peut citer les agents antioxydants ou anti-radicaux libres; les agents hydratants ou humectants tels que la glycérine et le collagène ; les agents filtres UV tels que la benzophénone. Ces actifs hydrosolubles peuvent être présents dans la composition finale en une quantité de 0 à 20%, de préférence 5 à 15%. en poids.

La présente invention concerne également un procédé de préparation d'une composition telle que définie précédemment, caractérisé par le fait que celle-ci est obtenue à partir d'un produit naturel ou d'un derivé de produit naturel apte à s'expanser, de la substance cosmétique ou dermatologique et éventuellement des autres ingrédients tels qu'énumérés ci-dessus, de préférence en présence d'eau, par mélange, malaxage et expansion dans un extrudeur bi-vis.

L'extrudeur utilisé pour le procédé de l'invention est choisi parmi les extrudeurs bi-vis tel que celui décrit dans la demande FR 94-00756.

Les matières premières sont introduites, à l'entrée de l'extrudeur bi-vis, dans la zone d'alimentation à température ambiante, de préférence à environ 20°C, puis sont amenées dans la zone de transport à une température, de préférence, à environ 50°C, puis sont malaxées et comprimées dans diverses zones de l'extrudeur maintenues à une température allant, préférentiellement, de 100 à 160°C ; la masse obtenue est transportée vers la sortie de l'extrudeur et extrudée au travers d'une filière pour y subir une expansion.

Pendant la phase de mélange, le produit naturel ou le dérivé de produit naturel apte à s'expanser, se gélatinise et forme, après extrusion, un réseau alvéolaire constituant la matrice des produits expansés finaux.

Un autre objet de l'invention consiste en de nouvelles compositions cosmétiques ou dermatologiques, caractérisées par le fait qu'elles sont constituées par une composition solide expansée telle que définie ci-dessus.

Ces compositions peuvent se présenter sous la forme de boudins, pellets ou flocons expansés ou bien être réduites à l'état de poudre. Elles peuvent être facilement réhydratables après immersion dans l'eau et susceptibles de reconstituer une formulation aqueuse liquide ou semi-liquide.

Ces compositions peuvent être des produits pour le maquillage. Elles peuvent être appliquées sur le visage soit directement soit par l'intermédiaire d'un outil de maquillage tel qu'un pinceau, une houpette ou un tampon applicateur. Elles peuvent être stockées à l'état sec et, au moment de l'utilisation, réhydratées après immersion dans l'eau pour reconstituer une formulation aqueuse de maquillage liquide ou semi-liquide telle qu'un fonds de teint.

Les compositions selon l'invention peuvent être des produits pour le soin et/ou l'hygiène de la peau, des muqueuses, du cuir chevelu ou des cheveux.

Elles peuvent se présenter sous forme de poudre et appliquées directement sur la peau, le cuir chevelu ou les cheveux comme par exemple un shampooing sec ou une poudre libre pour le soin du corps. Ces compositions peuvent également se présenter sous forme de poudre ou bien de boudins, pellets, de feuilles ou de flocons expansés, réhydratables après immersion dans l'eau de manière à reconstituer une formulation aqueuse pour le soin et/ou l'hygiène telle qu'une crème, un lait, un gel, un bain moussant, un shampooing.

Un autre objet de l'invention porte sur l'utilisation d'une composition solide expansée telle que définie ci-dessus dans et pour la fabrication d'une composition cosmétique ou dermatologique.

En particulier, l'invention concerne l'utilisation comme phase pulvérulente dans et pour la fabrication d'une composition de maquillage notamment sous forme de poudre compactée telle qu'un fard à joues ou un fard à paupières.

Bien entendu, l'homme de l'art veillera à choisir ce ou ces éventuels composés complémentaires et/ou leurs quantités de manière telle que les propriétés avantageuses attachés intrinsèquement à la composition conforme à l'invention ne soient pas, ou substantiellement pas, altérées par la ou les adjonctions envisagées.

Les exemples qui suivent servent à illustrer l'invention sans pour autant en limiter la portée.

### EXEMPLES

### Exemple 1 Shampooing sec sous forme de pellets à réhydrater

Le produit final a pour formulation suivante :

| | |
|---|---|
| - Farine de blé | 35,0% en poids |
| - Amidon de maïs | 35,0% en poids |
| - Silice vendue sous le nom SB700 par la Société MAPRECOS | 17,5% en poids |
| - Lauryl-éther sulfate de sodium | 12,5% en poids |

### MODE OPERATOIRE :

Les pellets sont obtenus par extrusion dans un extrudeur bi-vis. Les matières premières sont introduites à l'entrée de l'extrudeur à une température de 30°C. Elles sont ensuite amenées dans la zone de transport à une température de 50°C, puis sont malaxées et comprimées dans diverses zones de l'extrudeur maintenues à 120°C. La masse ainsi malaxée et comprimée, est transportée vers la sortie de l'extrudeur et extrudée au travers d'une filière de 5 mm de diamètre. La vitesse de rotation des vis est de 500 tours/minute. Les boudins obtenus à la sortie de la filière sont réduits sous forme de pellets de 3mm de diamètre au moyen d'un couteau granulateur en sortie de l'extrudeur.

### Exemple 2 Fonds de teint sous forme de poudre à réhydrater

Le produit final a pour formulation suivante :

| | |
|---|---|
| - Farine de blé | 40,0% en poids |
| - Huile de sésame | 5,0% en poids |
| - Talc | 25,0% en poids |
| - Silice vendue sous le nom SB700 par la Société MAPRECOS | 25,0% en poids |
| - Pigments | 5,0% en poids |

### MODE OPERATOIRE :

Les matières premières sont introduites à l'entrée d'un extrudeur bi-vis à une température de 30°C. Elles sont ensuite amenées dans la zone de transport à une température de 50°C, puis sont malaxées et comprimées dans diverses zones de l'extrudeur maintenues à 120°C. La masse ainsi malaxée et comprimée, est transportée vers la sortie de l'extrudeur et extrudée au travers d'une filière de 5 mm de diamètre. La vitesse de rotation des vis est de 500 tours/minute. Les boudins obtenus à la sortie de la filière sont réduits à l'état de poudre au moyen d'un broyeur à broches classique, placé à la sortie de l'extrudeur.

## Revendications

1. Utilisation comme produit cosmétique d'une composition solide expansée dont la matrice est constituée d'un réseau alvéolaire formé à partir d'un produit riche en amidon ou d'un dérivé de produit riche en amidon, apte à s'expanser par extrusion.

2. Utilisation selon la revendication 1, caractérisée par le fait que le produit riche en amidon est choisi parmi les farines de céréale ou de pomme de terre, les amidons purs, les amidons modifiés au niveau du rapport amylose/amylopectine, les amidons réticulés et les amidons modifiés par un groupe fonctionnel.

3. Utilisation selon la revendication 1 ou 2, caractérisée par le fait que la composition solide expansée contient au moins une charge minérale et/ou une charge organique.

4. Composition cosmétique ou dermatologique, caractérisée par le fait qu'elle est constituée par une composition solide expansée constituée d'un réseau alvéolaire formé à partir d'un produit riche en amidon ou d'un dérivé de produit riche en amidon, apte à s'expanser par extrusion et qu'elle contient au moins une substance cosmétique ou dermatologique.

5. Composition selon la revendication 4, caractérisée par le fait que le produit riche en amidon est choisi parmi les farines de céréale ou de pomme de terre, les amidons purs, les amidons modifiés au niveau du rapport amylose/amylopectine, les amidons réticulés et les amidons modifiés par un groupe fonctionnel.

6. Composition selon la revendication 4 ou 5 contenant au moins une charge minérale et/ou une charge organique.

7. Composition selon l'une quelconque des revendications 4 à 6, caractérisée par le fait qu'elle contient de 2 à 75% en poids de charge par rapport au poids total de la composition.

8. Composition selon l'une quelconque des revendications 4 à 7, caractérisée par le fait que lorsque la ou les charges ont une densité inférieure à 0,1 g.cm⁻³, elles sont présentes à raison de 2 à 20% en poids par rapport au poids total de la composition et lorsque la ou les charges ont une densité supérieure à 0,5 g.cm⁻³, elles sont présentes à raison de 10 à 75% en poids par rapport au poids total de la composition.

9. Composition selon l'une quelconque des revendications 4 à 8, caractérisée par le fait que la ou les charges sont choisies parmi les charges minérales ou organiques, de structure lamellaire ou sphérique, compactables ou difficilement compactables et leurs mélanges.

10. Composition selon la revendication 9, dans laquelle les charges de type minérales lamellaires sont choisies parmi les talcs ou silicates de magnésium hydratés, les micas ou aluminosilicates, les argiles telles que les séricites, le kaolin ou silicate d'aluminium hydraté, les nitrures de bore, les micatitanes.

11. Composition selon la revendication 9, dans laquelle les charges de type lamellaires organiques sont des poudres de polymères de tétrafluoroéthylène ou la lauroyl-lysine.

12. Composition selon la revendication 9, dans laquelle les charges de type sphériques minérales sont choisies parmi les oxydes de zinc et de titane, le carbonate de calcium précipité, le carbonate et l'hydro-carbonate de magnésium, la silice sphérique non poreuse, l'hydroxyapatite, les microsphères de silice à porosité ouverte ou creuses, éventuellement imprégnées d'un actif cosmétique et les microcapsules de verre ou de céramique.

13. Composition selon la revendication 9, dans laquelle les charges de type organiques sphériques sont choisies parmi les savons métalliques dérivés d'acides organiques carboxyliques ayant de 8 à 22 atomes de carbone, tels que le stéarate de zinc, de magnésium ou de lithium, le laurate de zinc, le myristate de magnésium ; les poudres de polymères synthétiques non expansés; les poudres de polymères synthétiques réticulés ou non, sphéronisées; les poudres de matériaux organiques d'origine naturelle, les microsphères microporeuses de polymères, éventuellement imprégnées par des actifs cosmétiques ; les microcapsules de polymères éventuellement réticulés.

14. Composition selon l'une quelconque des revendications 4 à 13, dans laquelle la matrice expansée représente de 25 à 98% en poids du poids total de la composition.

15. Composition selon la revendication 14, caractérisée par le fait que lorsque la ou les charges ont une densité inférieure à 0,1 g.cm⁻³, la matrice constituée d'un réseau alvéolaire amidonné représente de 80 à 98% en poids du poids total de la composition et lorsque la ou les charges ont une densité supérieure à 0,5 g.cm⁻³, la matrice représente de 25 à 90% en poids du poids total de la composition.

16. Composition selon l'une quelconque des revendications 4 à 15, dans laquelle la teneur en eau est inférieure à 5% en poids par rapport au poids total de la composition.

17. Composition selon la revendication 16, dans laquelle la teneur en eau varie de 1 à 2% en poids par rapport au poids total de la composition.

18. Composition selon l'une quelconque des revendications 4 à 17, caractérisée par le fait qu'elle contient en plus une phase grasse.

19. Composition selon la revendication 18, comprenant 2 à 30% en poids de phase grasse.

20. Composition selon l'une des revendications 18 et 19, dans laquelle la phase grasse comprend des huiles et/ou des cires d'origine animale, végétale, minérale ou synthétique, seules ou en mélanges.

21. Composition selon l'une quelconque des revendications 4 à 20, caractérisée par le fait qu'elle contient au moins une substance cosmétique ou dermatologique choisie dans le groupe constitué par les pigments, les tensio-actifs, les actifs liposolubles, les additifs lipososolubles habituellement utilisés en cosmétique, les anti-oxydants, les anti-radicaux libres, les hydratants, les humectants, les filtres solaires.

22. Composition selon l'une quelconque des revendications 4 à 21, caractérisée par le fait qu'elle se présente sous forme de boudin, pellet, feuille ou flocon expansé ou bien qu'elle est réduite à l'état de poudre.

23. Composition selon la revendication 22, caractérisée par le fait qu'elle est réhydratable après immersion dans l'eau et susceptible de reconstituer une formulation aqueuse liquide ou semi-liquide.

24. Composition selon l'une quelconque des revendications 22 et 23, caractérisée par le fait qu'elle est un produit pour le maquillage.

25. Composition selon l'une quelconque des revendications 22 à 24, caractérisée par le fait qu'elle est un produit pour le soin et/ou l'hygiène de la peau, des muqueuses, du cuir chevelu ou des cheveux.

26. Procédé de préparation de la composition telle que définie dans l'une quelconque des revendications 4 à 25, caractérisée par le fait qu'elle est obtenue à partir d'un produit riche en amidon ou d'un dérivé de produit riche en amidon, de la substance dermatologique ou cosmétique et éventuellement des autres constituants tels que définis dans les revendications précédentes, par mélange, malaxage, expansion et extrusion dans un extrudeur bi-vis.

27. Procédé selon la revendication 26, caractérisé par le fait que les matières premières sont introduites, à l'entrée de l'extrudeur, à température ambiante puis sont amenées dans la zone de transport à température d'environ 50°C, puis sont malaxées et comprimées dans diverses zones de l'extrudeur maintenues à une température allant de 100 à 160°C; la masse obtenue est transportée vers la sortie de l'extrudeur et extrudée au travers d'une filière pour y subir une expansion.

28. Utilisation comme phase pulvérulente d'une composition selon l'une quelconque des revendications 4 à 25, réduite à l'état de poudre, dans et pour la fabrication d'une composition pour le maquillage

## Claims

1. Use, as a cosmetic product, of an expanded solid composition whose matrix consists of an alveolar network formed from a starch-rich product or from a derivative of a starch-rich product, capable of being expanded by extrusion.

2. Use according to Claim 1, characterized in that the starch-rich product is chosen from cereal or potato flours, pure starches, starches modified in respect of the amylose/amylopectin ratio, crosslinked starches and starches modified by a functional group.

3. Use according to Claim 1 or 2, characterized in that the expanded solid composition contains at least one inorganic filler and/or one organic filler.

4. Cosmetic or dermatological composition, characterized in that it consists of an expanded solid composition consisting of an alveolar network formed from a starch-rich product or from a derivative of a starch-rich product, capable of being expanded by extrusion, and in that it contains at least one cosmetic or dermatological substance.

5. Use according to Claim 4, characterized in that the starch-rich product is chosen from cereal or potato flours, pure starches, starches modified in respect of the amylose/amylopectin ratio, crosslinked starches and starches modified with a functional group.

6. Composition according to Claim 4 or 5, containing at least one inorganic filler and/or an organic filler.

7. Composition according to any one of Claims 4 to 6, characterized in that it contains from 2 to 75 % by weight of filler relative to the total weight of the composition.

8. Composition according to any one of Claims 4 to 7, characterized in that when the filler(s) have a density lower than 0.1 g cm⁻³, they are present in a proportion of 2 to 20 % by weight relative to the total weight of the composition and, when the filler(s) have a density higher than 0.5 g cm⁻³, they are present in a proportion of 10 to 75 % by weight relative to the total weight of the composition.

9. Composition according to any one of Claims 4 to 8, characterized in that the filler(s) are chosen from inorganic or organic fillers of lamellar or spherical structure, which are compactable or difficult to compact and mixtures thereof.

10. Composition according to Claim 9, in which the fillers of lamellar inorganic type are chosen from talcs or hydrated magnesium silicates, micas or alumino-silicates, clays such as sericites, kaolin or hydrated aluminium silicate, boron nitrides and titanium micas.

11. Composition according to Claim 9, in which the fillers of organic lamellar type are tetrafluoroethylene polymer powders or lauroyllysine.

12. Composition according to Claim 9, in which the fillers of inorganic spherical type are chosen from zinc and titanium oxides, precipitated calcium carbonate, magnesium carbonate and hydrocarbonate, nonporous spherical silica, hydroxyapatite, open-porosity or hollow silica microspheres optionally impregnated with a cosmetic active agent and glass or ceramic microcapsules.

13. Composition according to Claim 9, in which the fillers of spherical organic type are chosen from metal soaps derived from organic carboxylic acids containing from 8 to 22 carbon atoms, such as zinc, magnesium or lithium stearate, zinc laurate, magnesium myristate, powdered unexpanded synthetic polymers, powdered crosslinked or non-crosslinked, spheronized synthetic polymers, powdered organic materials of natural origin, microporous polymer microspheres, optionally impregnated with cosmetic active agents, and optionally crosslinked polymer microcapsules.

14. Composition according to any one of Claims 4 to 13, in which the expanded matrix represents from 25 to 98 % by weight of the total weight of the composition.

15. Composition according to Claim 14, characterized in that, when the filler(s) have a density lower than 0.1 g cm⁻³, the matrix consisting of a starchy alveolar network represents from 80 to 98 % by weight of the total weight of the composition and, when the filler(s) have a density higher than 0.5 g cm⁻³, the matrix represents from 25 to 90 % by weight of the total weight of the composition.

16. Composition according to any one of Claims 4 to 15, in which the water content is lower than 5 % by weight relative to the total weight of the composition.

17. Composition according to Claim 16, in which the water content varies from 1 to 2 % by weight relative to the total weight of the composition.

18. Composition according to any one of Claims 4 to 17, characterized in that it additionally contains a fatty phase.

19. Composition according to Claim 18, including 2 to 30 % by weight of fatty phase.

20. Composition according to either of Claims 18 and 19, in which the fatty phase includes oils and/or waxes of animal, plant, mineral or synthetic origin, by themselves or as mixtures.

21. Composition according to any one of Claims 4 to 20, characterized in that it contains at least one cosmetic or dermatological substance chosen from the group consisting of pigments, surfactants, liposoluble active agents, liposoluble additives usually employed in cosmetics, antioxidants, anti-free-radical agents, hydrating agents, moisturizers and sunscreens.

22. Composition according to any one of Claims 4 to 21, characterized in that it is in the form of a roll, pellet, sheet or flake which is expanded or else in that it is reduced to powder form.

23. Composition according to Claim 22, characterized in that it is capable of being rehydrated after immersion in water and capable of reconstituting a liquid or semiliquid aqueous formulation.

24. Composition according to either of Claims 22 and 23, characterized in that it is a make-up product.

25. Composition according to any one of Claims 22 to 24, characterized in that it is a product for the care and/or hygiene of the skin, of the mucous membranes, of the scalp or of the hair.

26. Process for the preparation of the composition as defined in any one of Claims 4 to 25, characterized in that it is obtained from a starch-rich product or from a derivative of a starch-rich product, from the dermatological or cosmetic substance and optionally from the other constituents as defined in the preceding claims, by mixing, blending, expansion and extrusion in a twin-screw extruder.

27. Process according to Claim 26, characterized in that the raw materials are introduced at the entry of the extruder at ambient temperature and are then brought into the transport zone at a temperature of approximately 50°C and are then blended and compressed in various zones of the extruder which are maintained at a temperature ranging from 100 to 160°C; the mass obtained is conveyed towards the exit of the extruder and is extruded through a die in order to undergo expansion therein.

28. Use as pulverulent phase of a composition according to any one of Claims 4 to 25, which is reduced to powder form, in, and for the manufacture of, a make-up composition.

## Patentansprüche

1. Verwendung als kosmetisches Produkt einer expandierten festen Zusammensetzung, deren Matrix aus einem porigen Netzwerk besteht, das aus einem stärkereichen Produkt oder einem Derivat eines stärkereichen Produkts gebildet ist, das durch Extrusion expandiert werden kann.

2. Verwendung nach Anspruch 1, dadurch gekennzeichnet, daß das stärkereiche Produkt unter Getreidemehlen und Kartoffelmehl, reinen Stärken, Stärken, die bzgl. des Amylose/Amylopectin-Verhältnisses modifiziert sind, vernetzten Stärken und mit einer funktionellen Gruppe modifizierten Stärken ausgewählt wird.

3. Verwendung nach Anspruch 1 oder 2, dadurch gekennzeichnet, daß die expandierte feste Zusammensetzung mindestens einen anorganischen und/oder organischen Füllstoff enthält.

4. Kosmetische oder dermatologische Zusammensetzung, dadurch gekennzeichnet, daß sie aus einer expandierten festen Zusammensetzung besteht, die aus einem porigen Netzwerk besteht, das aus einem stärkereichen Produkt oder einem Derivat eines stärkereichen Produkts gebildet ist, das durch Extrusion expandiert werden kann, und daß sie mindestens eine kosmetische oder dermatologische Substanz enthält.

5. Zusammensetzung nach Anspruch 4, dadurch gekennzeichnet, daß das stärkereiche Produkt unter Getreidemehlen und Kartoffelmehl, reinen Stärken, Stärken, die hinsichtlich des Amylose/Amylopectin-Verhältnisses modifiziert sind, vernetzten Stärken und mit einer funktionellen Gruppe modifizierten Stärken ausgewählt ist.

6. Zusammensetzung nach Anspruch 4 oder 5, die mindestens einen anorganischen und/oder organischen Füllstoff enthält.

7. Zusammensetzung nach einem der Ansprüche 4 bis 6, dadurch gekennzeichnet, daß sie 2 bis 75 Gew.-% Füllstoff, bezogen auf das Gesamtgewicht der Zusammensetzung, enthält.

8. Zusammensetzung nach einem der Ansprüche 4 bis 7, dadurch gekennzeichnet, daß, wenn der oder die Füllstoffe eine Dichte unter 0,1 g·cm³ aufweisen, sie in einem Anteil von 2 bis 20 Gew.-%, bezogen auf das Gesamtgewicht der Zusammensetzung, enthalten sind, und daß, wenn der oder die Füllstoffe eine Dichte über 0,5 g·cm³ aufweisen, sie in einem Anteil von 10 bis 75 Gew.-%, bezogen auf das Gesamtgewicht der Zusammensetzung, enthalten sind.

9. Zusammensetzung nach einem der Ansprüche 4 bis 8, dadurch gekennzeichnet, daß der oder die Füllstoffe unter anorganischen und organischen Füllstoffen mit lamellarer oder kugelförmiger Struktur, die verdichtet werden können oder nur schwer verdichtet werden können, und deren Gemischen ausgewählt sind.

10. Zusammensetzung nach Anspruch 9, wobei die Füllstoffe vom Typ der lamellaren anorganischen Füllstoffe unter Talken oder hydratisierten Magnesiumsilicaten, Glimmern oder Alumosilicaten, Tonen, wie z.B. Sericiten, Kaolin oder hydratisiertem Aluminiumsilicat, Bornitriden und Titanglimmern ausgewählt sind.

11. Zusammensetzung nach Anspruch 9, wobei die Füllstoffe vom Typ der organischen lamellaren Füllstoffe Pulver aus Tetrafluorethylenpolymeren oder Lauroyllysin sind.

12. Zusammensetzung nach Anspruch 9, wobei die Füllstoffe vom Typ der anorganischen kugelförmigen Füllstoffe ausgewählt sind unter Zinkoxid und Titandioxid, gefälltem Calciumcarbonat, Magnesiumcarbonat und Magnesiumhydrogencarbonat, nichtporösem kugelförmigem Siliciumdioxid, Hydroxyapatit, offenporigen oder hohlen Mikrokügelchen aus Siliciumdioxid, die ggf. mit einem kosmetischen Wirkstoff imprägniert sind, und Mikrokapseln aus Glas oder Keramik.

13. Zusammensetzung nach Anspruch 9, wobei die Füllstoffe vom Typ der kugelförmigen organischen Füllstoffe ausgewählt sind unter Metallseifen, die von organischen Carbonsäuren mit 8 bis 22 Kohlenstoffatomen abgeleitet sind, wie z.B. Zink-, Magnesium- oder Lithiumstearat, Zinklaurat, Magnesiummyristat, Pulvern nicht expandierter synthetischer Polymer, Pulvern von ggf. vernetzten, zu Kügelchen geformten synthetischen Polymeren, Pulvern organischer Materialien natürlicher Herkunft, mikroporösen Mikrokügelchen von Polymeren, die ggf. mit kosmetischen Wirkstoffen imprägniert sind, Mikrokapseln aus ggf. vernetzten Polymeren.

14. Zusammensetzung nach einem der Ansprüche 4 bis 13, wobei die expandierte Matrix 25 bis 98 Gew.-% des Gesamtgewichts der Zusammensetzung ausmacht.

15. Zusammensetzung nach Anspruch 14, dadurch gekennzeichnet, daß, wenn der oder die Füllstoffe eine Dichte unter 0,1 g·cm³ aufweisen, die aus einem stärkehaltigen porigen Netzwerk bestehende Matrix 80 bis 98 Gew.-% des Gesamtgewichts der Zusammensetzung ausmacht und daß, wenn der oder die Füllstoffe eine Dichte über 0,5 g·cm³ aufweisen, die Matrix 25 bis 90 Gew.-% des Gesamtgewichts der Zusammensetzung ausmacht.

16. Zusammensetzung nach einem der Ansprüche 4 bis 15, wobei der Wassergehalt weniger als 5 Gew.-%, bezogen auf das Gesamtgewicht der Zusammensetzung, beträgt.

17. Zusammensetzung nach Anspruch 16, wobei der Wassergehalt von 1 bis 2 Gew.-%, bezogen auf das Gesamtgewicht der Zusammensetzung, variiert.

18. Zusammensetzung nach einem der Ansprüche 4 bis 17, dadurch gekennzeichnet, daß sie außerdem eine Fettphase enthält.

19. Zusammensetzung nach Anspruch 18, die 2 bis 30 Gew.-% Fettphase enthält.

20. Zusammensetzung nach einem der Ansprüche 18 und 19, wobei die Fettphase Öle und/oder Wachse tierischer, pflanzlicher, anorganischer oder synthetischer Herkunft einzeln oder in Form von Gemischen enthält.

21. Zusammensetzung nach einem der Ansprüche 4 bis 20, dadurch gekennzeichnet, daß sie mindestens eine kosmetische oder dermatologische Substanz enthält, die unter Pigmenten, grenzflächenaktiven Stoffen, fettlöslichen Wirkstoffen, fettlöslichen Zusätzen, die herkömmlicherweise in der Kosmetik verwendet, Antioxidantien, Mitteln gegen freie Radikale, Hydratisierungsmitteln, Feuchthaltemitteln, Lichtschutzfiltern, ausgewählt ist.

22. Zusammensetzung nach einem der Ansprüche 4 bis 21, dadurch gekennzeichnet, daß sie in Form von expandierten Strängen, Pellets, Blättchen oder Flocken vorliegt oder daß sie zu einem Pulver zerkleinert ist.

23. Zusammensetzung nach Anspruch 22, dadurch gekennzeichnet, daß sie durch Eintauchen in Wasser rehydratisierbar ist und geeignet ist, eine flüssige oder halbflüssige wäßrige Formulierung zu bilden.

24. Zusammensetzung nach einem der Ansprüche 22 und 23, dadurch gekennzeichnet, daß sie ein Schminkprodukt ist.

25. Zusammensetzung nach einem der Ansprüche 22 bis 24, dadurch gekennzeichnet, daß sie ein Produkt für die Pflege und/oder Reinigung der Haut, der Schleimhäute, der Kopfhaut oder der Haare ist.

26. Verfahren zur Herstellung der Zusammensetzung, wie sie in einem der Ansprüche 4 bis 25 definiert ist, dadurch gekennzeichnet, daß sie aus einem stärkereichen Produkt oder einem Derivat eines stärkereichen Produkts, der dermatologischen oder kosmetischen Substanz und ggf. weiteren Bestandteilen, die wie in den vorhergehenden Ansprüchen definiert sind, durch Mischen, Kneten, Expandieren und Extrudieren in einem Doppelschneckenextruder hergestellt wird.

27. Verfahren nach Anspruch 26, dadurch gekennzeichnet, daß die Ausgangsmaterialien am Einlaß des Extruders bei Umgebungstemperatur zugegeben werden, wonach sie in der Förderzone auf eine Temperatur von etwa 50 °C gebracht werden, woraufhin sie in verschiedenen, bei einer Temperatur von 100 bis 160 °C gehaltenen Zonen des Extruders geknetet und komprimiert werden und daß die erhaltene Masse zum Ausstoß des Extruders gefördert wird und unter Bewirkung einer Expansion durch eine Düse extrudiert wird.

28. Verwendung als pulverförmige Phase einer Zusammensetzung nach einem der Ansprüche 4 bis 25, die zu einem Pulver zerkleinert ist, in und für die Herstellung einer Zusammensetzung zum Schminken.
